**Europäisches Patentamt**

**European Patent Office**

**Office européen des brevets**

(19)

(11) Numéro de publication : **0 327 418 B1**

# (12) FASCICULE DE BREVET EUROPEEN

(45) Date de publication du fascicule du brevet :
**12.04.95 Bulletin 95/15**

(51) Int. Cl.$^6$ : **G01N 33/92**

(21) Numéro de dépôt : **89400142.9**

(22) Date de dépôt : **18.01.89**

(54) **Procédé immunométrique pour le dosage de particules de lipoprotéine, anticorps monoclonaux anti-Lp(a) et application au diagnostic de l'athérosclérose.**

(30) Priorité : **19.01.88 US 145597**
**24.02.88 FR 8802214**

(43) Date de publication de la demande :
**09.08.89 Bulletin 89/32**

(45) Mention de la délivrance du brevet :
**12.04.95 Bulletin 95/15**

(84) Etats contractants désignés :
**AT BE CH DE ES FR GB GR IT LI LU NL SE**

(56) Documents cités :
**BULL. ACAD. NATLE MED, vol. 169, no. 6, 04 juin 1985, Paris (FR); J.F. FRUCHART et al., pp. 719-728**
**CLINICAL CHEMISTRY, vol. 32, no. 8, août 1986, Winston-Salem, IL (US); S.G. YOUNG et al., pp. 1484-1490**
**BIOCHEMISTRY, vol. 26, 1987, Easton, PA (US); E. KOREN et al., pp. 2734-2740**
**CHEMICAL ABSTRACTS, vol. 102, no. 3, 21 janvier 1985, J.F. FRUCHART et al., p. 341, no. 20551h**
**JOURNAL OF CLINICAL CHEMISTRY & CLINICAL BIOCHEMISTRY, vol. 23, no. 9 , 1985; J.F. FRUCHART et al., pp. 619-620**
**CHEMICAL ABSTRACTS, vol. 107, no. 19, 09 novembre 1987, S. MARVOVINA et al., p. 351, no. 171876x**
**NEW COMPREHENSIVE BIOCHEMISTRY, vol. 14, Plasma Lipoprotein, 1987, Elsevier; pp. 129-152**
**JOURNAL OF LIPID RESEARCH, vol. 26, 1985; pp. 540-548**
**ARTERIOCLEROSIS, vol. 7, 1987; p. 505a**
**ACTUALITES D'ANGEIOLOGIE , 17e année 1992, XVII, no. 2; pp. 26-29**
**POUR LA SCIENCE, no. 175, mai 1992; pp. 40-47**
**SPECTRA BIOLOGIE, no. 92/1, janvier-février 1992; pp. 27-34**
**ARTERIOCLEROSIS, A Decade in Perspective, 1991; pp. 34-36**

(56) Documents cités :
**JOURNAL OF LIPID RESEARCH, vol. 30, 1989; pp. 1437-1443**

(73) Titulaire : **INSTITUT PASTEUR**
**28, rue du Docteur Roux**
**F-75724 Paris Cédex 15 (FR)**
Titulaire : **INSTITUT PASTEUR DE LILLE**
**1, rue du Professeur Calmette**
**BP 245**
**F-59019 Lille Cédex (FR)**

(72) Inventeur : **Fruchart, Jean-Charles**
**23 Domaine de la Hollande**
**F-59320 Ennetieres en Weppes (FR)**
Inventeur : **Vu Dac, Ngoc**
**22 rue du Colibri**
**F-59650 Villeneuve d'Ascq (FR)**

(74) Mandataire : **Ores, Irène et al**
**CABINET ORES**
**6, Avenue de Messine**
**F-75008 Paris (FR)**

## Description

La présente invention est relative à un procédé immunométrique pour le dosage de particules de lipoprotéine contenant à la fois de l'apolipoprotéine a (apo(a)) et de l'apolipoprotéine B (apoB), à des anticorps monoclonaux anti-lipoprotéine (a) (anti-Lp(a)) et à l'application de ce procédé au diagnostic de l'athérosclérose.

Les maladies cardio-vasculaires ischémiques constituent la première cause de mortalité dans les pays industrialisés. L'athérosclérose en est la principale affection. C'est une maladie générale et multifactorielle qui est accompagnée de deux complications graves, l'infarctus du myocarde et l'atteinte des artères cérébrales.

De nombreuses études épidémiologiques et génétiques ont montré le rôle important des lipides transportés par les lipoprotéines dans la pathogénèse de cette grave affection. En effet, il est aujourd'hui parfaitement démontré que les dyslipoprotéinémies constituent le facteur de risque majeur.

La recherche de marqueurs de risque biologiques de l'athérosclérose a fait l'objet d'importants travaux. Ainsi de nombreuses méthodes immunochimiques adaptées au dosage des apolipoprotéines, ont permis d'attribuer à certaines d'entre elles (apoA-I et B principalement), un pouvoir discriminant plus important que celui obtenu par un dosage classique des lipides (cholestérol et triglycérides).

Les particules de lipoprotéine contiennent généralement au moins une apolipoprotéine associée aux lipides constituant lesdites particules.

Certaines particules de lipoprotéine sont considérées comme athérogènes, notamment celles contenant de l'apoB, alors que d'autres sont considérées comme non athérogènes et mêmes protectrices (particules contenant de l'apoA).

La lipoprotéine(a) ou Lp(a) est une particule de lipoprotéine particulière qui fait partie des particules athérogènes. Découverte par Berg en 1963 (Acta Path. Microbiol. Scand., 1963, 59, 368 - 382), sa composition exacte a donné lieu pendant de nombreuses années à des résultats controversés. Elle a été considérée dans un premier temps comme un polymorphisme des LDL, notamment par sa composition en apoB. Actuellement, on connaît mieux cette lipoprotéine. Sa copule protéique est composée d'une apoB liée par un ou plusieurs ponts disulfures à une ou deux glycoprotéines spécifiques (apo(a)) dont la structure primaire a été récemment déterminée. Sa composition lipidique est très proche de celle des LDL. Bon nombre de ses aspects, génétiques, métaboliques et pathologiques, commencent à être mieux connus, mais son rôle et sa fonction dans l'organisme demeurent encore obscurs.

La lipoprotéine Lp(a) désigne la particule Lp(a), elle contient de l'apoprotéine B-100 (apo B-100), de l'apoprotéine (a) (apo(a)) et un noyau central constitué de lipides hydrophobes (esters de cholestérol (CE) et glycérides (G)) ; à la surface de ce noyau central, on trouve des phospholipides (PL), du cholestérol libre (CL) et les apoprotéines précitées. L'apoprotéine B-100 est commune aux LDL (Low Density Lipoproteins) et à la Lp(a) ; l'apoprotéine (a) a une structure proche de celle du plasminogène.

Dans la suite du texte, on désigne par Lp(a):B, la particule lipoprotéique contenant de l'apo(a) et de l'apoB ; on désigne par LpAI:B, la particule contenant de l'apoAI et de l'apoB, la LpCIII:B correspond à la particule contenant de l'apoCIII et de l'apoB, la LpAII:B correspond à la particule lipoprotéinique contenant de l'apoAII et de l'apoB et la LpE:B correspond à la particule contenant de l'apoE et de l'apoB.

Les techniques immunochimiques développées jusqu'à présent ne permettent que le dosage d'apolipoprotéines et non de particules de lipoprotéine. Or ne permet pas de prévoir à quel type de particules lipoprotéiques elles sont associées.

Les différentes techniques antérieures incluent notamment l'immunodiffusion, l'électroimmunoessai, l'immunonephelométrie, et les radioimmunoessais et l'ELISA.

En ce qui concerne l'apolipoprotéine A (apoAI ou apoAII), on peut citer en particulier la demande internationale PCT WO-A-86 05493 au nom de SCRIPPS CLINIC AND RESEARCH FOUNDATION ou l'article au nom de S. MARCOVINA et al, paru dans Adv. Exp. Med., Biol., 210 (Lipoproteins Atheroscler.), 1987, 63-71.

En ce qui concerne la particule de lipoprotéine (a), un certain nombre de méthodes de recherche et de dosage de la Lp(a) ont été proposées.

Parmi les méthodes non-immunologiques de séparation de la Lp(a) proposées dans l'Art antérieur, l'électro-phorèse sur gel de polyacrylamide donne les meilleurs résultats, toutefois, la sensibilité de cette méthode est faible et ne permet que la détection de concentrations élevées en Lp(a).

Les techniques immunochimiques développées pour l'analyse quantitative et qualitative, qui étaient basées sur l'utilisation d'anticorps polyclonaux hétérogènes ou d'anticorps monoclonaux, présentent des réactions immunologiques croisées avec le plasminogène comme cela a été démontré récemment.

G.M. FLESS et al. (1987, *Immunological cross reactivity of human plasminogen*, Arteriosclerosis, 7: 505a), par exemple, montrent qu'il existe un degré important d'homologie entre l'apo(a) et le plasminogène et, qu'en conséquence, les anti-sérums dirigés contre l'apo(a) des particules Lp(a) présentent des réactions croisées avec le plasminogène.

Bien que l'intérêt du dosage des particules de lipoprotéines ait été mise en valeur par FRUCHART et al. (Bull. Acad. Natl. Med., vol. 169, n° 6, 1985, 719-728 ; L. Clin. Chem. Biochem., vol. 23, n° 9, 1985, 619-620), toutefois aucun de ces documents ne résout le problème des réactions croisées rencontré avec les particules contenant de l'apo(a).

Parmi les méthodes de recherches immunologiques, on peut citer notamment, la technique d'immunodiffusion selon Ouchterlony (PROG. ALLERGY, 1958, 5, 1) dans laquelle l'antigène et l'anticorps diffusent dans un gel d'agarose pour former un arc de précipitation, est essentiellement une technique de dépistage, de même que la technique d'immunoélectrophorèse qui en est une variante et que la méthode d'immuno-électrosynérèse.

Les méthodes de dosage immunologiques spécifiques proposées, immunodiffusion radiale selon MANCINI (Immunochem., 1965, 2, 235), électroimmunodiffusion selon LAURELL (Scand. J. Clin. Lab. Invest., 1972, 29S, 124), immunonéphélométrie, radioimmunologie, utilisent des anticorps polyclonaux qui sont hétérogènes de par leur origine et qui, par surcroît, requièrent la mise en oeuvre de processus préliminaires d'adsorption pour éliminer la réactivité croisée avec l'apoB, comme mentionnée plus haut.

La Demanderesse a mis au point une première méthode de dosage de la lipoprotéine(a) par une méthode d'immunolatex. Cette technique permet d'éviter certains inconvénients de l'immunonéphélométrie et de l'électroimmunodiffusion tout en présentant une grande rapidité d'exécution, une haute sensibilité et une importante économie de réactifs.

Toutefois, cette méthode a l'inconvénient de présenter également des réactions croisées.

Une technique de dosage immunoenzymologique utilisant un anticorps anti-Lp(a) a également été proposée (cf. DUVIC et Coll. J. LIPID. RES., 1985, 26, 540). Il s'agit d'une méthode compétitive qui consiste à immobiliser l'antigène sur un support solide et à mettre en compétition cet antigène et l'antigène du sérum avec un anticorps monoclonal LHLP-1. Toutefois, l'inconvénient majeur de cette méthode est qu'il est nécessaire de pouvoir disposer de Lp(a) pure ; or, cette lipoprotéine est difficile à purifier et présente une certaine instabilité à l'état pur, ce qui a conduit à abandonner ce type de dosage.

La présente invention s'est en conséquence donné pour but de pourvoir à un procédé de dosage immunométrique spécifique des particules de lipoprotéine, qui répond mieux aux nécessités de la pratique que les procédés proposés dans l'Art antérieur, notamment en ce qu'il permet effectivement de quantifier les particules lipoprotéiniques (a) et qu'il ne présente pas de réactions croisées.

La présente invention a pour objet une méthode de dosage immunométrique de particules de lipoprotéine contenant à la fois de l'apolipoprotéine (a) (apo(a)) et de l'apolipoprotéine B (apoB), dans un fluide biologique, caractérisée en ce qu'elle comprend :
- la préparation d'un échantillon de fluide biologique à une dilution $\geqq$ à 1/40 ;
- la mise en contact dudit échantillon dilué avec deux anticorps différents, à savoir un premier anticorps choisi dans le groupe constitué par des anticorps anti-apo(a) monoclonaux ou oligoclonaux, présentant une faible affinité pour le plasminogène, exprimée par leur constante de dissociation, $K_D$, comprise entre $0,45 \times 10^{-7}$ M et $0,47 \times 10^{-7}$ M et une grande affinité pour l'apoprotéine(a), exprimée par leur constante de dissociation, $K_D$, comprise entre $0,87 \times 10^{-11}$M et $0,32 \times 10^{-10}$M, lesquels anticorps capturent la particule de lipoprotéine à doser et un deuxième anticorps anti-apoB complète marqué de manière appropriée et
- la détection de la présence des particules par une technique appropriée, notamment un enzymoimmunoessai (EIA), un radioimmunoessai (RIA), une réaction d'agglutination ou une mesure de fluorimétrie.

On entend par anticorps oligoclonal, un mélange d'anticorps monoclonaux.

Selon un mode de mise en oeuvre avantageux de la méthode conforme à l'invention, ledit premier anticorps est immobilisé sur un support solide approprié, notamment des plaques de microtitration ou des billes.

Selon un autre mode de mise en oeuvre avantageux de la méthode conforme à l'invention, le deuxième anticorps est un anticorps polyclonal anti-apoB.

Selon un autre mode de mise en oeuvre avantageux de la méthode conforme à l'invention, le deuxième anticorps est un anticorps monoclonal anti-apoB.

Selon encore un autre mode de mise en oeuvre avantageux de la méthode conforme à l'invention, ledit deuxième anticorps est un anticorps oligoclonal anti-apoB.

Conformément à l'invention, ledit deuxième anticorps est marqué par toute technique appropriée connue en soi, notamment à l'aide d'un produit de marquage choisi dans le groupe qui comprend les enzymes, les substances fluorescentes, les substances radioactives ou des billes de latex appropriées.

La présence des particules de lipoprotéine est alors révélée soit par le développement d'une réaction colorée en présence d'un substrat de l'enzyme utilisée, soit par la mesure de la fluorescence, soit par la mesure de la radioactivité ou soit par le développement d'une réaction d'agglutination.

Conformément à l'invention, lorsque le premier anticorps est un anticorps anti-apo(a) et le deuxième anticorps est un anticorps anti-apoB, la particule détectée est la Lp(a):B.

La présente invention a également pour objet des anticorps anti-Lp(a), caractérisés en ce qu'ils sont constitués par des anticorps monoclonaux spécifiques de l'apoprotéine(a) (apo(a)), présentant une faible affinité pour le Plasminogène, exprimée par leur constante de dissociation, $K_D$, comprise entre $0,45 \times 10^{-7}$ M et $0,47 \times 10^{-7}$ M et une grande affinité pour l'apoprotéine(a), exprimée par leur constante de dissociation, $K_D$, comprise entre $0,87 \times 10^{-11}$M et $0,32 \times 10^{-10}$M.

Selon un mode de réalisation avantageux de la présente invention, lesdits anticorps monoclonaux sont obtenus par clonage d'hybridomes résultant de la fusion de cellules myélomateuses appropriées et de cellules spléniques murines immunisées par un antigène constitué par une Lp(a) d'origine humaine, en mettant en oeuvre une technique de clonage appropriée et par sélection des hybrides produisant des anticorps monoclonaux spécifiques de l'apoprotéine(a) (apo(a)), présentant une faible affinité pour le plasminogène, exprimée par leur constante de dissociation, $K_D$, comprise entre $0,45 \times 10^{-7}$ M et $0,47 \times 10^{-7}$ M et une grande affinité pour l'apoprotéine(a), exprimée par leur constante de dissociation, $K_D$, comprise entre $0,87 \times 10^{-11}$M et $0,32 \times 10^{-10}$M, et en ce que lesdits anticorps monoclonaux sont des immunoglobulines de type $IgG_1$.

La présente invention a, de plus, pour objet l'application de la méthode de dosage immunoenzymométrique conforme à l'invention au dépistage de l'athérosclérose.

La présente invention a, en outre, pour objet un kit ou coffret, prêt à l'emploi, pour la mise en oeuvre du procédé de dosage de particules de lipoprotéine et l'application dudit procédé au dépistage de l'athérosclérose, caractérisé en ce qu'il comprend :

- des doses appropriées d'un premier anticorps choisi dans le groupe constitué par des anticorps monoclonaux et oligoclonaux dirigés contre l'apolipoprotéine(a) ;
- des doses appropriées d'un deuxième anticorps anti-apoB complète, marqué de manière appropriée ;
- un sérum standard ;
- des quantités utiles de tampons appropriés pour la mise en oeuvre de ladite détection ; et
- éventuellement des moyens de révélation de la présence de particules de lipoprotéine.

Selon un autre mode de réalisation avantageux du kit conforme à la présente invention, le premier anticorps est immobilisé sur un support solide approprié, notamment des plaques de microtitration ou des billes.

Selon un autre mode de réalisation, le deuxième anticorps est un anticorps polyclonal anti-apoB complète.

Selon un autre mode de réalisation du kit conforme à l'invention, le deuxième anticorps est un anticorps monoclonal anti-apoB complète.

Selon un autre mode de réalisation du kit conforme à l'invention, le deuxième anticorps est un anticorps oligoclonal anti-apoB complète.

Selon un autre mode de réalisation avantageux du kit, le deuxième anticorps est couplé à un système enzymatique approprié.

Selon une disposition avantageuse de ce mode de réalisation, lorsque le deuxième anticorps est couplé à un système enzymatique, ledit kit comprend comme moyen de révélation de la présence de particules de lipoprotéine, une quantité appropriée de substrat convenable.

Selon encore un autre mode de réalisation du kit, le deuxième anticorps est couplé à une substance fluorescente appropriée.

En ce cas, la révélation est réalisée par détection de la fluorescence.

Selon encore un autre mode de réalisation du kit, le deuxième anticorps est couplé à des billes de latex.

En ce cas, la révélation est réalisée par l'apparition d'une réaction d'agglutination.

**Exemple 1: Obtention des échantillons sanguins.**

Les échantillons de sang sont obtenus par ponction veineuse chez des donneurs sains à jeun et collectés dans des tubes sous vide contenant de l'EDTA. Après addition de conservateurs (concentrations finales : EDTA 0,27 mmol/l, acide aminocaproïque 0,9 mmol/l, chloramphénicol 0,6 mmol/l et glutathion 0,3 mmol/l) et centrifugation pour séparer les cellules du plasma, les échantillons de plasma sont conservés à 4°C et doivent être utilisés dans les 48 heures ou sont congelés à -20°C.

**Exemple 2 : Isolement et Purification des particules de Lp(a).**

Une fraction riche en Lp(a) est isolée à partir d'un pool de plasma Lp(a)+ par ultracentrifugation, le plasma étant ajusté à une densité comprise entre 1,06 - 1,12 Kg/l par du bromure de potassium solide, puis centrifugé deux fois 24 heures à 100 000g et à 4°C dans une ultracentrifugeuse Beckman® L8-70 avec un rotor 50 Ti.

Pour la purification, une colonne de Sépharose CL 6B de dimension 100 x 2,6 cm est utilisée.

Après étalonnage à l'aide d'un kit de masse moléculaire, on procède à la purification.

La solution tampon d'élution est composée de Tris 0,1 M, NaCl 0,15 M, $Na_2$ EDTA 1mM, pH 8,2.

80 mg d'une fraction enrichie en Lp(a) par ultracentrifugation sont déposés.

L'élution est menée à + 4°C avec un débit de 8 ml/h au delà de 24 heures. La collecte s'effectue par fractions de 5 ml.

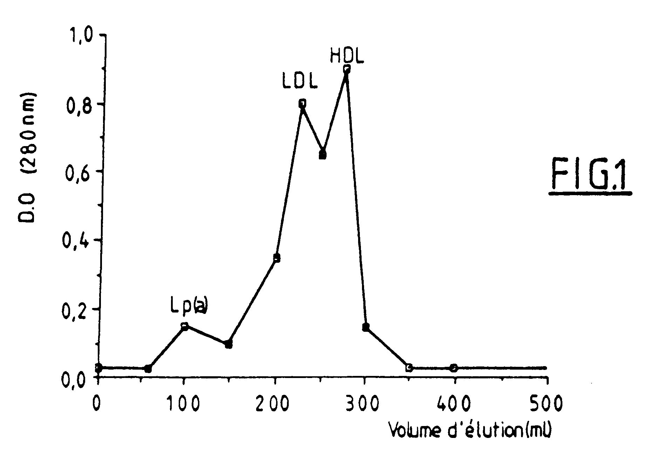

On filtre à travers un filtre millipore 0,22 μm. Sur la figure 1, on a en abscisse le volume d'élution en ml et en ordonnée la densité optique à 280 nm ; trois pics sont obtenus.

Le pic 1 donne des arcs de précipitation contre des antisérums anti-Lp(a) et anti-apoB, le pic 2 uniquement contre l'antisérum anti-apoB (LDL) et le pic 3 contre l'antiapoAI (HDL).

11 mg de Lp(a):B sont obtenus à partir du pic 1. Ces particules de Lp(a):B purifiées contiennent 30 % de protéines et 60 % de lipides.

**Exemple 3 : Préparation des anticorps monoclonaux anti-apo(a) conformes à l'invention :**

A. Purification de l'apo(a).

Une fraction de la Lp(a) enrichie conformément à l'exemple 2 est traitée comme suit :

On clive la Lp(a) au niveau des ponts disulfures, dans un tampon Tris 10 mM NaCl 0,05 M $Na_2$EDTA 1mM et $NaN_3$ 0,02 % pH 7,6 par addition de dithiothreitol (DTT) sous forme solide et de manière à obtenir une concentration finale de 10 mM.

Les solutions obtenues sont incubées à 37°C pendant trois jours et dialysées pendant une nuit à 4°C contre le même tampon sans DTT.

La solution est alors chromatographiée sur une colonne héparine sépharose® (Pharmacia). Deux pics sont élués, le premier pic correspond à l'apo(a) ; le deuxième pic est une particule LDL-like.

B. Production des anticorps monoclonaux anti-Lp(a).

1°) Immunisation :

Des souris mâles Balb/C sont immunisées par injection intrapéritonéale de 100 μg de Lp(a) mélangés à 300 μl d'adjuvant complet de Freund. Cette injection est répétée pour chaque souris 21 jours après, mais avec de l'adjuvant incomplet de Freund et ainsi de suite pendant 4 mois. Les souris sont saignées pour vérifier les réponses immunologiques avant de procéder à l'hybridation cellulaire.

2°) Obtention des anticorps monoclonaux :

- Fusion :

La souris répondant contre la Lp(a), ayant subi au préalable une injection intrapéritonéale de rappel au jour J-10, puis une dernière en intra-veineuse au jour J-3 est saignée. L'immunsérum est récupéré. Il sera aliquoté puis congelé afin de servir de témoin positif lors du criblage. La rate de l'animal est prélevée.

La rate, prélevée stérilement sur boîte de Pétri, est dilacérée. Les cellules spléniques obtenues sont fusionnées avec des cellules myélomateuses $SP_2/0$, au moyen de PEG-DMSO, selon le protocole décrit par Kohler et Milstein.

Les proportions cellules spléniques/cellules myélomateuses sont de 10/1.

Après élimination du PEG, la totalité des cellules est reprise dans du milieu sélectif HAT, et répartie dans des boîtes de culture de 96 puits, à raison de 25 000 cellules par puits dans deux gouttes de milieu HAT. Les boîtes sont mises à l'étuve à 37°C en atmosphère humide et en présence de 5 % de dioxyde de carbone.

Les cellules sont cultivées en présence de milieu sélectif HAT, puis après 15 jours en présence de milieu HT, puis finalement en présence de milieu A, jusqu'à disparition des cellules myélomateuses non hybridées. Les puits sont testés par une technique de criblage immunoenzymatique.

- Clonage :

Les puits positifs sont clonés par dilution limite.

Une vingtaine de puits de culture ont ainsi été détectés. Trois puits présentant une sécrétion d'immunoglobulines dirigées uniquement contre les LDL ont également été retenus.

Les cellules sécrétrices en culture sont mises en suspension, puis un prélèvement est effectué et dilué au 1/10e dans un tube stérile contenant du milieu A. On procède ensuite au comptage à l'aide d'une cellule de Fuchs sur un prélèvement d'une dilution au 1/100e. Le nombre de cellules est ramené à 20 cellules par ml de milieu A. Les cellules sont ensuite réparties dans des boîtes de culture de 96 puits contenant des cellules péritonéales de souris (cellules nourricières), préparées 24 à 48 heures auparavant, à raison de 50 µl par puits, soit en théorie 1 cellule pour 50 µl. Les clones positifs anti-Lp(a) sont clonés une seconde fois, voire une troisième fois. Les clones positifs sont ensuite multipliés en masse puis conservés dans l'azote liquide ou mis en production d'ascite.

Sur une vingtaine de puits en culture, produisant des anticorps dirigés contre la Lp(a), seulement 4 clones stables ont pu être obtenus et conservés. Ce sont les clones K01, K06, K07 et K09. A partir de ces clones, on a procédé à la production de liquide d'ascite et enfin d'anticorps monoclonaux. La classe de chaque anticorps est déterminée par immunodiffusion avec des immunsérums commerciaux spécifiques. Les quatres clones produisent des IgG, aisément purifiables par chromatographie sur protéine-A Sépharose®.

- Production de liquide d'ascite.

Deux méthodes ont été utilisées :
. Les cellules hybrides issues d'un clone sont injectées intra-péritonéalement chez des souris Balb/C ayant reçu 10 jours auparavant 0,5 ml de pristane ; environ $5.10^6$ cellules hybrides sont injectées par souris. Les cellules sont préalablement lavées par du milieu Dulbecco afin d'éliminer le sérum de veau foetal. Ces souris ont été auparavant déprimées du point de vue immunitaire par injection de pristane, afin d'éliminer le risque de rejet.

Les cellules hybrides développent une ascite chez les souris ; ce liquide d'ascite est riche en anticorps monoclonaux.
. La seconde méthode est plus adaptée pour une production en masse. Dans une première étape, on développe par injection sous-cutanée de 2 à $3.10^6$ cellules, une tumeur dans un délai de 15 jours. Cette tumeur est le siège d'une multiplication cellulaire très importante. Après prélèvement, la tumeur est broyée, les cellules sont lavées par du milieu Dulbecco puis injectées par voie intra-péritonéale à des souris Balb/c non "pristanisées". Une tumeur peut permettre l'injection de 8 à 10 souris. Une autre souris étant dans le même temps injectée par voie sous-cutanée afin d'entretenir le cycle de production.

Dans les deux méthodes, le liquide d'ascite est recueilli puis centrifugé à 3000 tr/mn (10 mn), seul le surnageant est recueilli. L'addition de $10^{-4}$ M de PMSF permet d'inhiber l'action de protéases. Le stockage s'effectue à -20°C. Pour chaque clone K01, K06, K07 et K09 un volume important de liquide d'ascite a été récupéré.

3°) Purification des anticorps monoclonaux :

- Précipitation au sulfate d'ammonium

Les ascites sont additionnées d'une solution de sulfate d'ammonium à 50 % (P/V).

Deux précipitations successives sont effectuées. Une partie de l'albumine ainsi que l'hémoglobine sont ainsi éliminés. Le dernier précipité est repris dans un volume minimum de solution de chlorure de sodium 0,15 M et dialysé une nuit contre la même solution.

- Purification sur colonne d'affinité

La seconde étape est la purification proprement dite des IgG par chromatographie sur protéine A-Sépharose®. Une quantité optimale de protéines ressolubilisées est déposée sur une colonne équilibrée par une solution tampon de phosphate de sodium 0,10 pH 8, puis incubée au minimum 30 mn à température ambiante. La colonne est ensuite lavée avec la solution tampon d'équilibre puis éluée avec une solution tampon citrate-citrique de sodium 0,1 M pH 3. Toutes les classes d'IgG sont éluées. L'éluat est immédiatement neutralisé par une solution 1 M de phosphate di-potassique ($K_2HPO_4$). La colonne est rééquilibrée par le tampon initial. La fraction d'IgG est dialysée contre une solution de PBS 0,1 M pH 7,2 avant stockage à -20°C.

Les anticorps monoclonaux subissent un test rapide de spécificité par la technique ELISA contre l'apo(a), la Lp(a), les LDL et les HDL, puis sont caractérisés et utilisés pour le dosage immunoenzymatique de la particule Lp(a).

Le tableau I ci-après montre le contrôle de spécificité des anticorps monoclonaux anti-Lp(a) par ELISA directe :

## TABLEAU I

| AcM<br>Ag | D.O. 492 nm | | | |
|---|---|---|---|---|
| | KO1 | KO6 | KO7 | KO9 |
| Apo(a) | 1,0 | 1,1 | 0,9 | 1,1 |
| Lp(a) | 1,1 | 1,5 | 1,0 | 1,4 |
| LDL | 0 | 0 | 0 | 0 |
| HDL | 0 | 0 | 0 | 0 |

**Exemple 4 : Caractérisation des anticorps monoclonaux anti-apo(a) : leur affinité vis-à-vis de la Lp(a):B et du plasminogène.**

Ces anticorps montrent une réaction croisée avec le plasminogène.

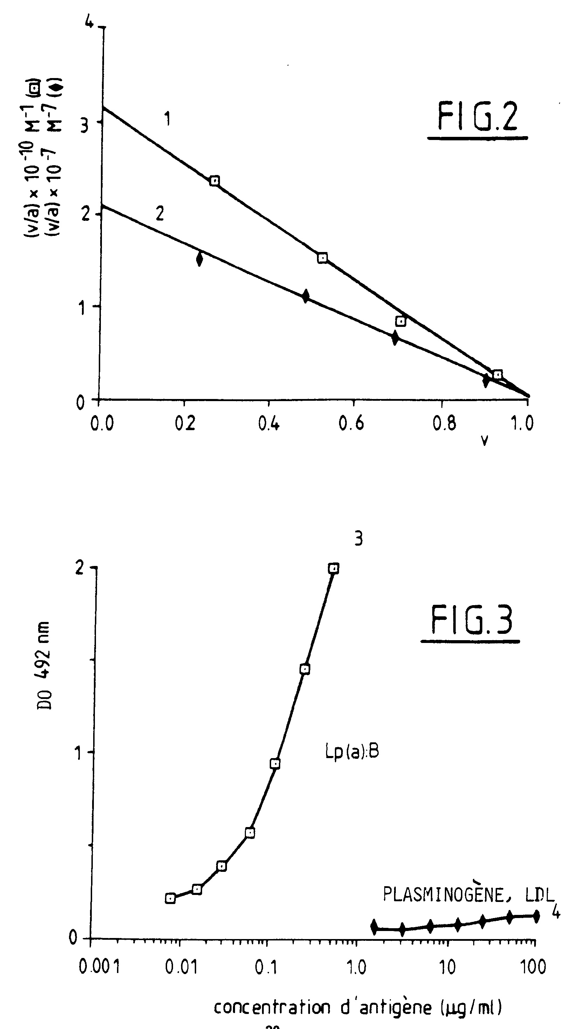

Les constantes de dissociation ont été déterminées comme décrit par FRIGUET et al. (J. Immunol. Methods, 1985, 77, 305-319). 0,1 ml d'anticorps (30 ng/ml de K07 ou de K09) sont incubés avec 0,1 ml de Lp(a):B ou de plasminogène à différentes concentrations. Après 15 heures d'incubation à la température ambiante, 0,1 ml du mélange est transféré et incubé pendant deux heures à 37°C dans les puits de plaques de microtitration préalablement enduites avec de la Lp(a):B purifiée. L'anticorps lié est détecté à l'aide d'une immunoglobuline de lapin anti-souris marquée à la peroxydase. La constante de dissociation $K_D$ est calculée au moyen de l'équation de SCATCHARD. On observe des différences importantes entre l'affinité des anticorps monoclonaux K07 et K09 vis-à-vis de la Lp(a):B et du plasminogène. Les résultats sont présentés dans le tableau II ci-après ainsi que sur la figure 2.

## TABLEAU II

| Ag          AcM | $K_D$ | | |
|---|---|---|---|
| | KO7 | KO9 | K07 + K09 |
| Lp(a):B | $0,87 \times 10^{-11}M$ | $0,29 \times 10^{-10}M$ | $0,32 \times 10^{-10}M$ |
| Plasminogène | $0,45 \times 10^{-7}M$ | $0,47 \times 10^{-7}M$ | $0,47 \times 10^{-7}M$ |

La figure 2 représente les constantes de dissociation calculées par l'équation de SCATCHARD modifiée ; la courbe 1 représente la liaison de la Lp(a):B avec un mélange d'anticorps monoclonaux anti-apo(a) (K07 + K09) et la courbe 2 représente la liaison du plasminogène avec un mélange d'anticorps monoclonaux anti-apo(a) (K07 + K09) ; v correspond à la fraction d'anticorps lié et a à la concentration d'antigène libre.

Ces anticorps monoclonaux anti-apo(a) présentent une affinité importante pour l'apo(a).

**Exemple 5 : Préparation des anticorps polyclonaux anti-apoB complète :**

Ces anticorps sont préparés chez le lapin, et la fraction IgG est purifiée par précipitation au sulfate de sodium suivie d'une chromatographie d'affinité.

Les anticorps polyclonaux anti-apoB purifiés sont couplés avec de la peroxydase de manière à former un conjugué.

7

**Exemple 6 : Préparation de l'anticorps oligoclonal anti-apoB complète.**

Les anticorps monoclonaux contre l'apoB sont obtenus par fusion de cellules myélomateuses $SP_2/O$ et de cellules spléniques obtenues à partir de souris immunisées avec des LDL. Les anticorps dénommés B04, B05, B06, B07, B18 et B19 sont spécifiques de l'apoB et ne réagissent avec aucune autre protéine du plasma. Leurs épitopes sont localisés sur les fragments de la thrombine T1/T3 de l'apo-B100.

Le mélange d'anticorps monoclonaux anti-apoB sont couplés avec de la peroxydase de manière à former un conjugué.

**Exemple 7 : Dosage immuno-enzymologique sélectif de la particule Lp(a):B, le premier anticorps étant un mélange des anticorps anti-apo(a) monoclonaux K07 et K09 (anticorps oligoclonal) et le deuxième anticorps étant un mélange d'anticorps monoclonaux (ou anticorps oligoclonal) anti-apoB (B04, B05, B06, B07, B18, B19), marqué à la peroxydase.**

Le dosage immunoenzymologique selon l'invention utilise la technique sandwich décrite dans le Brevet Hybritech n° 24 87983 du 3 août 1981.

- Courbe d'étalonnage.

Le standard primaire correspond à des particules de Lp(a):B conformes à l'exemple 2, exprimant des antigènes apo(a) et apoB (figure 3); il est utilisé pour étalonner un pool de 50 plasmas du commerce. La zone linéaire de dosage de la courbe standard s'étend de 0,06 à 0,40 µg/ml. La figure 3 représente la courbe d'étalonnage des particules de Lp(a):B purifiées et comporte en abscisse la concentration en antigènes en µg/ml (particules de Lp(a):B : courbe 3 ; plasminogène ou LDL : courbe 4) et en ordonnée l'absorbance à 492 nm. La densité optique d'un échantillon négatif correspond à 0,050.

- Dosage proprement dit :

. des plaques de microtitration en polystyrène (Costar 3590) sont enduites la veille au soir, à température ambiante, avec 0,1 ml d'un mélange d'anticorps monoclonaux anti-apo(a) conforme à l'exemple 3 (mélange d'anticorps monoclonaux K07 et K09, dans un rapport molaire 1:1), à une concentration de 20 µg/ml dans 0,1 M de PBS ;

. le pool de plasma standard est dilué de manière à ce que la concentration en particules de Lp(a):B soit comprise entre 0,06 et 0,40 µg/ml et les échantillons de plasma à doser sont dilués au 200ème, au 800ème et au 3 200éme dans du PBS-BSA 1% ;

. les plaques sont rincées 4 fois avec du PBS, avant d'additionner 100 µl d'échantillon ou de standard sur les parois des puits, l'incubation étant alors poursuivie à 37°C pendant 150 mn ;

. après un rinçage avec du PBS, 0,1 ml de conjugué (antiapoB/peroxydase à 1 mg/ml) dilué au 1/1000 dans du PBS-BSA à 1 %, est ajouté et incubé à 37°C pendant 120 minutes ;

. les puits sont à nouveau rincés avec du PBS, puis on ajoute O,1 ml d'une solution d'un substrat de la peroxydase (O-phénylènediamine 3 mg/ml et $H_2O_2$ concentré 0,64 µl/ml dans un tampon citrate-phosphate 0,1 M, pH 5,6), qui au bout de 30 mn, à l'obscurité, développe une réaction colorée. La réaction est interrompue par l'addition de 0,1 ml de HCl 1 N et l'absorbance est déterminée à 492 nm.

**Exemple 8 : Spécificité du procédé conforme à l'invention vis-à-vis de la Lp(a):B.**

a) Interférence avec le plasminogène.

La spécificité des anticorps a été établie en mesurant l'affinité du mélange d'anticorps monoclonaux anti-apo(a) K07 et K09 vis-à-vis de l'apo(a) de la particule Lp(a):B et du plasminogène (exemple 4).

Cependant, pour montrer la grande spécificité du procédé, il convient d'étudier l'éventuelle interférence du plasminogène.

Pour estimer l'interférence dans les conditions du procédé de dosage conforme à l'invention, une inhibition compétitive est réalisée en présence de la Lp(a):B purifiée et du plasminogène purifié. Les essais de compétition sont réalisés pour une concentration constante de Lp(a):B et une série de concentrations différentes de plasminogène ; on ajoute et on incube 0,05 ml de Lp(a) à 0,06 mg/dl et 0,05 ml de plasminogène à des concentrations comprises entre 0,1 et 80 mg/dl à des puits préalablement enduits d'un mélange d'anticorps anti-apo(a) K07 et K09.

On détermine ensuite la quantité de Lp(a):B liée comme décrit précédemment (voir exemple 7).

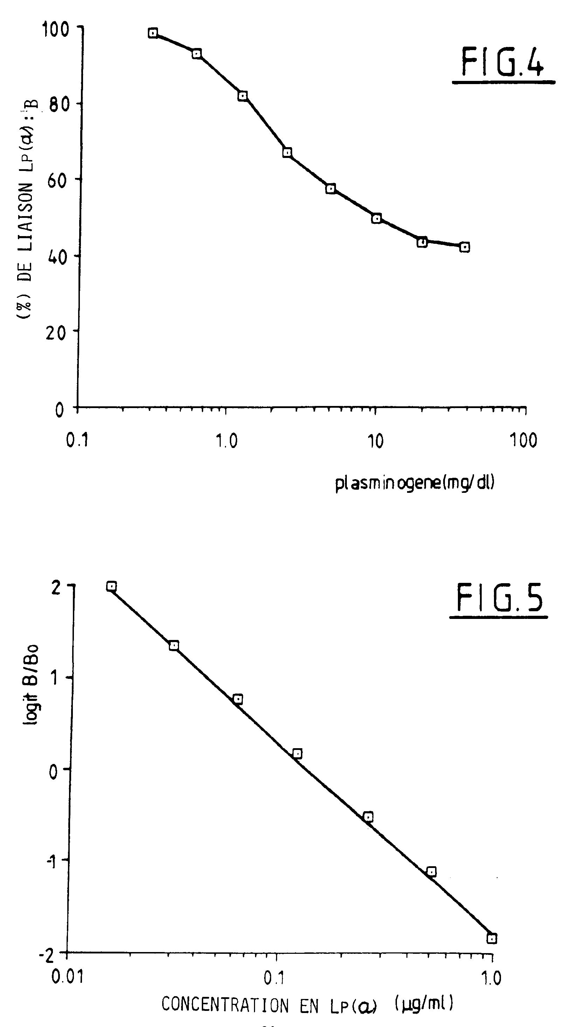

La figure 4 représente l'éventuelle interférence du plasminogène dans les conditions du procédé conforme à l'invention de détermination des particules de Lp(a):B.

Les résultats sont exprimés par B (présence de plasminogène)/ B0 (absence de plasminogène).

La figure 4 comporte un abscisse la concentration en plasminogène (mg/dl) et en ordonnée le pourcentage de particules Lp(a):B liées. Comme le montre cette figure, le plasminogène à haute concentration (40 mg/dl) pourrait inhiber jusqu'à 56 % de la liaison de la Lp(a):B. Cependant à une concentration de 1,25 mg/dl, l'inhibition est d'environ 18 %. Comme ce taux de plasminogène correspond généralement à la dilution au 1/40 du plasma, qui contient des taux de plasminogène relativement constants (47 ± 7 mg/dl) et comme dans la pratique on n'utilise que cette dilution pour la détermination de taux plasmatiques très faibles de Lp(a):B (< à 0,1 mg/dl), il apparaît que l'interférence du plasminogène est évitée dans le procédé conforme à l'invention.

De plus, comme montré dans l'exemple 3, une différence nette est observée dans l'affinité des anticorps K07 et K09 pour la Lp(a):B et du plasminogène, figure 1.

b) Spécificité des anticorps anti-apo(a) : comparaison avec le standard secondaire par l'étude de l'expression des épitopes reconnus par les anticorps monoclonaux K07 et K09 sur les Lp(a):B de différents plasmas et sur le standard secondaire.

Pour diminuer l'éventuelle influence de l'hétérogénéité de l'apo(a) sur l'expression d'un épitope, on utilise un mélange d'anticorps monoclonaux K07 et K09. L'expression concomitante de ces épitopes est déterminée sur dix plasmas différents ayant des concentrations en Lp(a):B comprises entre 1 et 40 mg/dl. La méthode d'inhibition compétitive telle que décrite dans l'exemple 4 permet également de déterminer l'expression des épitopes des anticorps monoclonaux K07 et K09. Les pentes des courbes d'inhibition compétitive sont similaires et exprimées en fonction du logit de $B/B_0$ (anticorps lié/anticorps libre) et sont comprises entre - 1,8 et - 2,15 avec une valeur moyenne de - 2; la pente typique d'un pool de plasma Lp(a):B est de - 2,05 (figure 5). Ces résultats suggèrent que les épitopes K07 et K09 sont exprimés similairement sur les Lp(a) de différents individus.

La figure 5 comprend en abscisse la concentration en Lp(a):B exprimée en $\mu$g/ml et en ordonnée le logit de $B/B_0$.

Pour déterminer l'immunoréactivité des Lp(a):B individuelles, la Lp(a):B a été mesurée dans dix plasmas ayant une concentration en Lp(a):B comprise entre 1 et 40 mg/dl. Les courbes ELISA obtenues, exprimées par le logit de la densité optique à 450 nm, présentent des pentes comprises entre 1,85 et 2,30 avec une valeur moyenne de 2,15. La pente du standard secondaire est de 2,12.

Pour estimer la précision de l'essai, différentes quantités de Lp(a):B purifiées (5, 20, 50 $\mu$g) ont été ajoutées à trois plasmas présentant des taux différents en Lp(a):B (1, 10, 30 mg/dl). La précision est de 85 à 100 %.

c) Etude de la répétabilité et de la reproductibilité du procédé.

La précision de la méthode a également été explorée en mesurant neuf plasmas dix fois sur la même plaque de microtitation, de manière à obtenir le coefficient de variation (CV) intra-essai (répétabilité) (valeur moyenne de CV intra-essai = 4,7 %). Ces plasmas ont également été testés trois jours de suite de manière à obtenir le coefficient de variation inter-essai (reproductibilité) (valeur moyenne de CV inter-essai = 9,6 %).

L'effet du stockage à -20° sur les Lp(a):B plasmatiques a été étudié sur les mêmes neufs plasmas à des jours différents. Le coefficient de variation est de 9,8 %.

Les différents coefficients de variation sont faibles et montrent donc la précision et l'intérêt du procédé conforme à l'invention.

**Revendications**

**Revendications pour les Etats contractants suivants : AT, BE, CH, DE, FR, GB, IT, LI, LU, NL, SE**

1. Méthode de dosage immunométrique de particules de lipoprotéine contenant à la fois de l'apolipoprotéine (a) (apo(a)) et de l'apolipoprotéine B (apoB), dans un fluide biologique, caractérisée en ce qu'elle comprend :
   - la préparation d'un échantillon de fluide biologique à une dilution $\geq$ à 1/40 ;
   - la mise en contact dudit échantillon dilué avec deux anticorps différents, à savoir un premier anticorps choisi dans le groupe constitué par des anticorps anti-apo(a) monoclonaux ou oligoclonaux, présentant une faible affinité pour le plasminogène, exprimée par leur constante de dissociation, $K_D$, comprise entre $0,45 \times 10^{-7}$ M et $0,47 \times 10^{-7}$ M et une grande affinité pour l'apoprotéine(a), exprimée par leur constante de dissociation, $K_D$, comprise entre $0,87 \times 10^{-11}$M et $0,32 \times 10^{-10}$M, lesquels anticorps capturent la particule de lipoprotéine à doser et un deuxième anticorps anti-apoB complète marqué de manière appropriée et

- la détection de la présence des particules par une technique appropriée, notamment un enzymoimmunoessai (EIA), un radioimmunoessai (RIA), une réaction d'agglutination ou une mesure de fluorimétrie.

2. Méthode selon la revendication 1, caractérisée en ce que ledit premier anticorps est immobilisé sur un support solide approprié, notamment des plaques de microtitration ou des billes.

3. Méthode selon la revendication 1 ou la revendication 2, caractérisée en ce que le deuxième anticorps est un anticorps polyclonal anti-apoB.

4. Méthode selon la revendication 1 ou la revendication 2, caractérisée en ce que le deuxième anticorps est un anticorps monoclonal anti-apoB.

5. Méthode selon la revendication 1 ou la revendication 2, caractérisée en ce que ledit deuxième anticorps est un anticorps oligoclonal anti-apoB.

6. Méthode selon l'une quelconque des revendications 1 à 5, caractérisée en ce que ledit deuxième anticorps est marqué par toute technique appropriée connue en soi notamment à l'aide d'un produit de marquage choisi dans le groupe qui comprend les enzymes, les substances fluorescentes, les substances radioactives ou des billes de latex appropriées.

7. Anticorps anti-Lp(a), caractérisés en ce qu'ils sont constitués par des anticorps monoclonaux spécifiques de l'apoprotéine(a) (apo(a)), présentant une faible affinité pour le plasminogène, exprimée par leur constante de dissociation, $K_D$, comprise entre $0,45 \times 10^{-7}$ M et $0,47 \times 10^{-7}$ M et une grande affinité pour l'apoprotéine(a), exprimée par leur constante de dissociation, $K_D$, comprise entre $0,87 \times 10^{-11}$M et $0,32 \times 10^{-10}$M.

8. Anticorps selon la revendication 7, caractérisés en ce qu'ils sont obtenus par clonage d'hybridomes résultant de la fusion de cellules myélomateuses appropriées et de cellules spléniques murines immunisées par un antigène constitué par une Lp(a) d'origine humaine, en mettant en oeuvre une technique de clonage appropriée et par sélection des hybrides produisant des anticorps monoclonaux spécifiques de l'apoprotéine(a) (apo(a)), présentant une faible affinité pour le plasminogène, exprimée par leur constante de dissociation, $K_D$, comprise entre $0,45 \times 10^{-7}$ M et $0,47 \times 10^{-7}$ M et une grande affinité pour l'apoprotéine(a), exprimée par leur constante de dissociation, $K_D$, comprise entre $0,87 \times 10^{-11}$M et $0,32 \times 10^{-10}$M, et en ce que lesdits anticorps monoclonaux sont des immunoglobulines de type $IgG_1$.

9. Application de la méthode de dosage selon l'une quelconque des revendications 1 à 6 au dépistage de l'athérosclérose.

10. Kit ou coffret, prêt à l'emploi, pour la mise en oeuvre du procédé de dosage de particules de lipoprotéine selon l'une quelconque des revendications 1 à 6, et l'application dudit procédé au dépistage de l'athérosclérose selon la revendication 9, caractérisé en ce qu'il comprend :
- des doses appropriées d'un premier anticorps choisi dans le groupe constitué par des anticorps monoclonaux et oligoclonaux dirigés contre l'apolipoprotéine(a) ;
- des doses appropriées d'un deuxième anticorps anti-apoB complète, marqué de manière appropriée ;
- un sérum standard ;
- des quantités utiles de tampons appropriés pour la mise en oeuvre de ladite détection ; et
- éventuellement des moyens de révélation de la présence de particules de lipoprotéine.

11. Kit selon la revendication 10, caractérisé en ce que le premier anticorps est immobilisé sur un support solide approprié, notamment des plaques de microtitration ou des billes.

12. Kit selon la revendication 10, caractérisé en ce que ledit premier anticorps monoclonal est un anticorps selon la revendication 7 ou la revendication 8.

**13.** Kit selon l'une quelconque des revendications 10 à 12, caractérisé en ce que le deuxième anticorps est un anticorps polyclonal anti-apoB complète.

**14.** Kit selon l'une quelconque des revendications 10 à 12, caractérisé en ce que le deuxième anticorps est un anticorps monoclonal anti-apoB complète.

**15.** Kit selon l'une quelconque des revendications 10 à 12, caractérisé en ce que le deuxième anticorps est un anticorps oligoclonal anti-apoB complète.

**16.** Kit selon l'une quelconque des revendications 10 à 15, caractérisé en ce que le deuxième anticorps est couplé à un système enzymatique approprié.

**17.** Kit selon la revendication 16, caractérisé en ce que, lorsque le deuxième anticorps est couplé à un système enzymatique, ledit kit comprend comme moyen de révélation de la présence de particules de lipoprotéine, une quantité appropriée de substrat convenable.

**18.** Kit selon l'une quelconque des revendications 10 à 15, caractérisé en ce que le deuxième anticorps est couplé à une substance fluorescente appropriée.

**19.** Kit selon l'une quelconque des revendications 10 à 15, caractérisé en ce que le deuxième anticorps est couplé à des billes de latex.

**Revendications pour les Etats contractants suivants : ES, GR**

**1.** Méthode de dosage immunométrique de particules de lipoprotéine contenant à la fois de l'apolipoprotéine (a) (apo(a)) et de l'apolipoprotéine B (apoB), dans un fluide biologique, caractérisée en ce qu'elle comprend :
  - la préparation d'un échantillon de fluide biologique à une dilution $\geqq$ à 1/40 ;
  - la mise en contact dudit échantillon dilué avec deux anticorps différents, à savoir un premier anticorps choisi dans le groupe constitué par des anticorps anti-apo(a) monoclonaux ou oligoclonaux, présentant une faible affinité pour le plasminogène, exprimée par leur constante de dissociation, $K_D$, comprise entre $0,45 \times 10^{-7}$ M et $0,47 \times 10^{-7}$ M et une grande affinité pour l'apoprotéine(a), exprimée par leur constante de dissociation, $K_D$, comprise entre $0,87 \times 10^{-11}$M et $0,32 \times 10^{-10}$M, lesquels anticorps capturent la particule de lipoprotéine à doser et un deuxième anticorps anti-apoB complète marqué de manière appropriée et
  - la détection de la présence des particules par une technique appropriée, notamment un enzymoimmunoessai (EIA), un radioimmunoessai (RIA), une réaction d'agglutination ou une mesure de fluorimétrie.

**2.** Méthode selon la revendication 1, caractérisée en ce que ledit premier anticorps est immobilisé sur un support solide approprié, notamment des plaques de microtitration ou des billes.

**3.** Méthode selon la revendication 1 ou la revendication 2, caractérisée en ce que le deuxième anticorps est un anticorps polyclonal anti-apoB.

**4.** Méthode selon la revendication 1 ou la revendication 2, caractérisée en ce que le deuxième anticorps est un anticorps monoclonal anti-apoB.

**5.** Méthode selon la revendication 1 ou la revendication 2, caractérisée en ce que ledit deuxième anticorps est un anticorps oligoclonal anti-apoB.

**6.** Méthode selon l'une quelconque des revendications 1 à 5, caractérisée en ce que ledit deuxième anticorps est marqué par toute technique appropriée connue en soi notamment à l'aide d'un produit de marquage choisi dans le groupe qui comprend les enzymes, les substances fluorescentes, les substances radioactives ou des billes de latex appropriées.

**7.** Procédé de préparation d'anticorps anti-Lp(a), caractérisés en ce qu'ils sont obtenus par immunisation appropriée par un antigène constitué par une Lp(a) d'origine humaine, lesquels anticorps sont constitués par des anticorps monoclonaux spécifiques de l'apoprotéine(a) (apo(a)), présentant une faible affinité pour le plasminogène, exprimée par leur constante de dissociation, $K_D$, comprise entre 0,45 x $10^{-7}$ M et 0,47 x $10^{-7}$ M et une grande affinité pour l'apoprotéine(a), exprimée par leur constante de dissociation, $K_D$, comprise entre 0,87 x $10^{-11}$M et 0,32 x $10^{-10}$M.

8. Procédé de préparation des anticorps selon la revendication 7, caractérisés en ce qu'ils sont obtenus par clonage d'hybridomes résultant de la fusion de cellules myélomateuses appropriées et de cellules spléniques murines immunisées par un antigène constitué par une Lp(a) d'origine humaine, en mettant en oeuvre une technique de clonage appropriée et par sélection des hybrides produisant des anticorps monoclonaux spécifiques de l'apoprotéine(a) (apo(a)), présentant une faible affinité pour le plasminogène, exprimée par leur constante de dissociation, $K_D$, comprise entre 0,45 x $10^{-7}$ M et 0,47 x $10^{-7}$ M et une grande affinité pour l'apoprotéine(a), exprimée par leur constante de dissociation, $K_D$, comprise entre 0,87 x $10^{-11}$M et 0,32 x $10^{-10}$M, et en ce que lesdits anticorps monoclonaux sont des immunoglobulines de type $IgG_1$.

9. Application de la méthode de dosage selon l'une quelconque des revendications 1 à 6 au dépistage de l'athérosclérose.

10. Kit ou coffret, prêt à l'emploi, pour la mise en oeuvre du procédé de dosage de particules de lipoprotéine selon l'une quelconque des revendications 1 à 6, et l'application dudit procédé au dépistage de l'athérosclérose selon la revendication 9, caractérisé en ce qu'il comprend :
    - des doses appropriées d'un premier anticorps choisi dans le groupe constitué par des anticorps monoclonaux et oligoclonaux dirigés contre l'apolipoprotéine(a) ;
    - des doses appropriées d'un deuxième anticorps anti-apoB complète, marqué de manière appropriée ;
    - un sérum standard ;
    - des quantités utiles de tampons appropriés pour la mise en oeuvre de ladite détection ; et
    - éventuellement des moyens de révélation de la présence de particules de lipoprotéine.

11. Kit selon la revendication 10, caractérisé en ce que le premier anticorps est immobilisé sur un support solide approprié, notamment des plaques de microtitration ou des billes.

12. Kit selon la revendication 10, caractérisé en ce que ledit premier anticorps monoclonal est un anticorps selon la revendication 7 ou la revendication 8.

13. Kit selon l'une quelconque des revendications 10 à 12, caractérisé en ce que le deuxième anticorps est un anticorps polyclonal anti-apoB complète.

14. Kit selon l'une quelconque des revendications 10 à 12, caractérisé en ce que le deuxième anticorps est un anticorps monoclonal anti-apoB complète.

15. Kit selon l'une quelconque des revendications 10 à 12, caractérisé en ce que le deuxième anticorps est un anticorps oligoclonal anti-apoB complète.

16. Kit selon l'une quelconque des revendications 10 à 15, caractérisé en ce que le deuxième anticorps est couplé à un système enzymatique approprié.

17. Kit selon la revendication 16, caractérisé en ce que, lorsque le deuxième anticorps est couplé à un système enzymatique, ledit kit comprend comme moyen de révélation de la présence de particules de lipoprotéine, une quantité appropriée de substrat convenable.

18. Kit selon l'une quelconque des revendications 10 à 15, caractérisé en ce que le deuxième anticorps est couplé à une substance fluorescente appropriée.

19. Kit selon l'une quelconque des revendications 10 à 15, caractérisé en ce que le deuxième anticorps est couplé à des billes de latex.

**Patentansprüche**

**Patentansprüche für folgende Vertragsstaaten : AT, BE, CH, DE, FR, GB, IT, LI, LU, NL, SE**

1. Verfahren zur immunometrischen Bestimmung von Lipoproteinteilchen in einer biologischen Flüssigkeit, welche gleichzeitig Apolipoprotein (a) [Apo (a)] und Apolipoprotein B [Apo B] enthalten, dadurch gekennzeichnet, daß es umfaßt:
   - die Herstellung einer biologischen Flüssigkeitsprobe in einer Verdünnung von $\geqq 1/40$;
   - das Inkontaktbringen der verdünnten Probe mit zwei unterschiedlichen Antikörpern, nämlich einem ersten Antikörper, ausgewählt aus der Gruppe bestehend aus: monoklonalen oder oligoklonalen anti-Apo (a) Antikörpern, die eine schwache Affinität zu Plasminogen aufweisen, ausgedrückt durch ihre Dissoziationskonstante $K_D$, die zwischen $0,45 \times 10^{-7}$ M und $0,47 \times 10^{-7}$ M beträgt, und eine hohe Affinität zum Apoprotein (a) aufweisen, ausgedrückt durch ihre Dissoziationskonstante $K_D$, die zwischen $0,87 \times 10^{-11}$ M und $0,32 \times 10^{-10}$ M beträgt, wobei die Antikörper das zu bestimmende Lipoproteinteilchen und einen zweiten anti-Apo B vollständig Antikörper, der auf geeignete Art markiert ist, fangen, und
   - den Nachweis des Vorliegens der Teilchen mittels einer geeigneten Technik, insbesondere einem Enzymimmuntest (EIA), einem Radioimmuntest (RIA), einer Agglutinationsreaktion oder einer fluorimetrischen Messung.

2. Verfahren nach Anspruch 1, dadurch gekennzeichnet, daß der erste Antikörper auf einem geeigneten festen Träger immobilisiert wird, insbesondere auf Mikrotiterplatten oder Kügelchen.

3. Verfahren nach Anspruch 1 oder 2, dadurch gekennzeichnet, daß der zweite Antikörper ein polyklonaler anti-Apo B Antikörper ist.

4. Verfahren nach Anspruch 1 oder 2, dadurch gekennzeichnet, daß der zweite Antikörper ein monoklonaler anti-Apo B Antikörper ist.

5. Verfahren nach Anspruch 1 oder 2, dadurch gekennzeichnet, daß der zweite Antikörper ein oligoklonaler anti-Apo B Antikörper ist.

6. Verfahren nach einem der Ansprüche 1 bis 5, dadurch gekennzeichnet, daß der zweite Antikörper durch sämtliche geeigneten an sich bekannten Techniken markiert wird, insbesondere mit Hilfe eines Markierungsproduktes, ausgewählt aus der Gruppe, welche umfaßt: Enzyme, fluoreszierende Substanzen, radioaktive Substanzen oder geeignete Latexkügelchen.

7. Anti-Lp (a) Antikörper, dadurch gekennzeichnet, daß sie gebildet sind aus monoklonalen Antikörpern, spezifisch für Apoprotein (a) [Apo (a)], eine schwache Affinität für Plasminogen aufweisen, ausgedrückt durch ihre Dissoziationskonstante $K_D$, die zwischen $0,45 \times 10^{-7}$ M und $0,47 \times 10^{-7}$ M beträgt, und eine hohe Affinität zum Apoprotein (a) aufweisen, ausgedrückt durch ihre Dissoziationskonstante $K_D$, die zwischen $0,87 \times 10^{-11}$ M und $0,32 \times 10^{-10}$ M beträgt.

8. Antikörper nach Anspruch 7, dadurch gekennzeichnet, daß man sie durch Klonieren von Hybridomen erhält, welche das Ergebnis einer Fusion von geeigneten Myelomzellen und Milzzellen von Mäusen sind, welche mit einem Antigen immunisiert wurden, das aus einem Lp (a) menschlichen Ursprungs gebildet ist, indem man eine geeignete Klonierungstechnik sowie eine Selektion der Hybriden, welche die für das Apoprotein (a) [Apo (a)] spezifischen monoklonalen Antikörper herstellen, durchführt, welche eine schwache Affinität für Plasminogen aufweisen, ausgedrückt durch ihre Dissoziationskonstante $K_D$, die zwischen $0,45 \times 10^{-7}$ M und $0,47 \times 10^{-7}$ M beträgt, und eine hohe Affinität zum Apoprotein (a) aufweisen, ausgedrückt durch ihre Dissoziationskonstante $K_D$, die zwischen $0,87 \times 10^{-11}$ M und $0,32 \times 10^{-10}$ M beträgt, und wobei die monoklonalen Antikörper Immunglobuline vom Typ $IgG_1$ sind.

9. Verwendung des Verfahrens zur Bestimmung nach einem der Ansprüche 1 bis 6, zur Feststellung von Arteriosklerose.

10. Gebrauchsfertiger Kit oder Packung zur Durchführung des Verfahrens zur Bestimmung von Lipoproteinteilchen nach einem der Ansprüche 1 bis 6, und der Anwendung des Verfahrens zur Feststellung der Arteriosklerose nach Anspruch 9, dadurch gekennzeichnet, daß er umfaßt:

- geeignete Mengen eines ersten Antikörpers, ausgewählt aus der Gruppe bestehend aus: Gegen Apolipoprotein (a) gerichtete monoklonale und oligoklonale Antikörper;
- geeignete Mengen eines zweiten Antikörpers anti-Apo B vollständig, in geeigneter Weise markiert;
- ein Standardserum;
- zweckdienliche Puffermengen, die geeignet sind, um die Bestimmung durchzuführen; und
- gegebenenfalls Mittel zur Erkennbarmachung des Vorliegens von Lipoproteinteilchen.

11. Kit nach Anspruch 10, dadurch gekennzeichnet, daß, der erste Antikörper auf einem geeigneten festen Träger immobilisiert ist, insbesondere Mikrotiterplatten oder Kügelchen.

12. Kit nach Anspruch 10, dadurch gekennzeichnet, daß der erste monoklonale Antikörper ein Antikörper nach Anspruch 7 oder Anspruch 8 ist.

13. Kit nach einem der Ansprüche 10 bis 12, dadurch gekennzeichnet, daß der zweite Antikörper ein polyklonaler anti-Apo B vollständig Antikörper ist.

14. Kit nach einem der Ansprüche 10 bis 12, dadurch gekennzeichnet, daß der zweite Antikörper ein monoklonaler anti-Apo B vollständig Antikörper ist.

15. Kit nach einem der Ansprüche 10 bis 12, dadurch gekennzeichnet, daß der zweite Antikörper ein oligoklonaler anti-Apo B vollständig Antikörper ist.

16. Kit nach einem der Ansprüche 10 bis 15, dadurch gekennzeichnet, daß der zweite Antikörper an ein geeignetes enzymatisches System gekoppelt ist.

17. Kit nach Anspruch 16, dadurch gekennzeichnet, daß, wenn der zweite Antikörper an ein enzymatisches System gekoppelt ist, der Kit als ein Mittel zur Erkennbarmachung des Vorliegens von Lipoproteinteilchen, eine geeignete Menge eines passenden Substrates umfaßt.

18. Kit nach einem der Ansprüche 10 bis 15, dadurch gekennzeichnet, daß der zweite Antikörper an eine geeignete fluoreszierende Substanz gekoppelt ist.

19. Kit nach einem der Ansprüche 10 bis 15, dadurch gekennzeichnet, daß der zweite Antikörper an Latexkügelchen gekoppelt ist.

**Patentansprüche für folgende Vertragsstaaten : ES, GR**

1. Verfahren zur immunometrischen Bestimmung von Lipoproteinteilchen in einer biologischen Flüssigkeit, welche gleichzeitig Apolipoprotein (a) [Apo (a)] und Apolipoprotein B [Apo B] enthalten, dadurch gekennzeichnet, daß es umfaßt:
   - die Herstellung einer biologischen Flüssigkeitsprobe in einer Verdünnung von $\geqq 1/40$;
   - das Inkontaktbringen der verdünnten Probe mit zwei unterschiedlichen Antikörpern, nämlich einem ersten Antikörper, ausgewählt aus der Gruppe bestehend aus: monoklonalen oder oligoklonalen anti-Apo (a) Antikörpern, die eine schwache Affinität zu Plasminogen aufweisen, ausgedrückt durch ihre Dissoziationskonstante $K_D$, die zwischen $0,45 \times 10^{-7}$ M und $0,47 \times 10^{-7}$ M beträgt, und eine hohe Affinität zum Apoprotein (a) aufweisen, ausgedrückt durch ihre Dissoziationskonstante $K_D$, die zwischen $0,87 \times 10^{-11}$ M und $0,32 \times 10^{-10}$ M beträgt, wobei die Antikörper das zu bestimmende Lipoproteinteilchen und einen zweiten anti-Apo B vollständig Antikörper, der auf geeignete Art markiert ist, fangen, und
   - den Nachweis des Vorliegens der Teilchen mittels einer geeigneten Technik, insbesondere einem Enzymimmuntest (EIA), einem Radioimmuntest (RIA), einer Agglutinationsreaktion oder einer fluorimetrischen Messung.

2. Verfahren nach Anspruch 1, dadurch gekennzeichnet, daß der erste Antikörper auf einem geeigneten festen Träger immobilisiert wird, insbesondere auf Mikrotiterplatten oder Kügelchen.

3. Verfahren nach Anspruch 1 oder 2, dadurch gekennzeichnet, daß der zweite Antikörper ein polyklonaler anti-Apo B Antikörper ist.

4. Verfahren nach Anspruch 1 oder 2, dadurch gekennzeichnet, daß der zweite Antikörper ein monoklonaler anti-Apo B Antikörper ist.

5. Verfahren nach Anspruch 1 oder 2, dadurch gekennzeichnet, daß der zweite Antikörper ein oligoklonaler anti-Apo B Antikörper ist.

6. Verfahren nach einem der Ansprüche 1 bis 5, dadurch gekennzeichnet, daß der zweite Antikörper durch sämtliche geeigneten an sich bekannten Techniken markiert wird, insbesondere mit Hilfe eines Markierungsproduktes, ausgewählt aus der Gruppe, welche umfaßt: Enzyme, fluoreszierende Substanzen, radioaktive Substanzen oder geeignete Latexkügelchen.

7. Verfahren zur Herstellung von Anti-Lp (a) Antikörpern, dadurch gekennzeichnet, daß man sie durch eine geeignete Immunisierung durch ein Antigen erhält, welches aus einem Lp (a) menschlichen Ursprungs gebildet ist, wobei die Antikörper gebildet sind aus monoklonalen Antikörpern, spezifisch für Apoprotein (a) [Apo (a)], eine schwache Affinität für Plasminogen aufweisen, ausgedrückt durch ihre Dissoziationskonstante $K_D$, die zwischen $0,45 \times 10^{-7}$ M und $0,47 \times 10^{-7}$ M beträgt, und eine hohe Affinität zum Apoprotein (a) aufweisen, ausgedrückt durch ihre Dissoziationskonstante $K_D$, die zwischen $0,87 \times 10^{-11}$ M und $0,32 \times 10^{-10}$ M beträgt.

8. Verfahren zur Herstellung von Antikörpern nach Anspruch 7, dadurch gekennzeichnet, daß man sie durch Klonieren von Hybridomen erhält, welche das Ergebnis einer Fusion von geeigneten Myelomzellen und Milzzellen von Mäusen sind, welche mit einem Antigen immunisiert wurden, das aus einem Lp (a) menschlichen Ursprungs gebildet ist, indem man eine geeignete Klonierungstechnik sowie eine Selektion der Hybriden, welche die für das Apoprotein (a) [Apo (a)] spezifischen monoklonalen Antikörper herstellen, durchführt, welche eine schwache Affinität für Plasminogen aufweisen, ausgedrückt durch ihre Dissoziationskonstante $K_D$, die zwischen $0,45 \times 10^{-7}$ M und $0,47 \times 10^{-7}$ M beträgt, und eine hohe Affinität zum Apoprotein (a) aufweisen, ausgedrückt durch ihre Dissoziationskonstante $K_D$, die zwischen $0,87 \times 10^{-11}$ M und $0,32 \times 10^{-10}$ M beträgt, und wobei die monoklonalen Antikörper Immunglobuline vom Typ $IgG_1$ sind.

9. Verwendung des Verfahrens zur Bestimmung nach einem der Ansprüche 1 bis 6, zur Feststellung von Arteriosklerose.

10. Gebrauchsfertiger Kit oder Packung zur Durchführung des Verfahrens zur Bestimmung von Lipoproteinteilchen nach einem der Ansprüche 1 bis 6, und der Anwendung des Verfahrens zur Feststellung der Arteriosklerose nach Anspruch 9, dadurch gekennzeichnet, daß er umfaßt:
    - geeignete Mengen eines ersten Antikörpers, ausgewählt aus der Gruppe bestehend aus: Gegen Apolipoprotein (a) gerichtete monoklonale und oligoklonale Antikörper;
    - geeignete Mengen eines zweiten Antikörpers anti-Apo B vollständig, in geeigneter Weise markiert;
    - ein Standardserum;
    - zweckdienliche Puffermengen, die geeignet sind, um die Bestimmung durchzuführen; und
    - gegebenenfalls Mittel zur Erkennbarmachung des Vorliegens von Lipoproteinteilchen.

11. Kit nach Anspruch 10, dadurch gekennzeichnet, daß der erste Antikörper auf einem geeigneten festen Träger immobilisiert ist, insbesondere Mikrotiterplatten oder Kügelchen.

12. Kit nach Anspruch 10, dadurch gekennzeichnet, daß der erste monoklonale Antikörper ein Antikörper nach Anspruch 7 oder Anspruch 8 ist.

13. Kit nach einem der Ansprüche 10 bis 12, dadurch gekennzeichnet, daß der zweite Antikörper ein polyklonaler anti-Apo B vollständig Antikörper ist.

14. Kit nach einem der Ansprüche 10 bis 12, dadurch gekennzeichnet, daß der zweite Antikörper ein monoklonaler anti-Apo B vollständig Antikörper ist.

15. Kit nach einem der Ansprüche 10 bis 12, dadurch gekennzeichnet, daß der zweite Antikörper ein oligoklonaler anti-Apo B vollständig Antikörper ist.

16. Kit nach einem der Ansprüche 10 bis 15, dadurch gekennzeichnet, daß der zweite Antikörper an ein geeignetes enzymatisches System gekoppelt ist.

17. Kit nach Anspruch 16, dadurch gekennzeichnet, daß, wenn der zweite Antikörper an ein enzymatisches System gekoppelt ist, der Kit als ein Mittel zur Erkennbarmachung des Vorliegens von Lipoproteinteilchen, eine geeignete Menge eines passenden Substrates umfaßt.

18. Kit nach einem der Ansprüche 10 bis 15, dadurch gekennzeichnet, daß der zweite Antikörper an eine geeignete fluoreszierende Substanz gekoppelt ist.

19. Kit nach einem der Ansprüche 10 bis 15, dadurch gekennzeichnet, daß der zweite Antikörper an Latexkügelchen gekoppelt ist.

## Claims

### Claims for the following Contracting States : AT, BE, CH, DE, FR, GB, IT, LI, LU, NL, SE

1. Method of immunometric assay of lipoprotein particles containing both apolipoprotein (a) (apo(a)) and apolipoprotein B (apoB), in a biological fluid, characterized in that it comprises:
   - preparation of a sample of biological fluid at a dilution $\geqq 1/40$;
   - bringing the said diluted sample into contact with two different antibodies, namely a first antibody chosen from the group consisting of monoclonal or oligoclonal anti-apo(a) antibodies possessing a low affinity for plasminogen, expressed by their dissociation constant $K_D$ of between $0.45 \times 10^{-7}$M and $0.47 \times 10^{-7}$M, and a high affinity for apoprotein(a), expressed by their dissociation constant $K_D$ of between $0.87 \times 10^{-11}$M and $0.32 \times 10^{-10}$M, which antibodies capture the lipoprotein particle to be assayed, and a second, suitably labelled anti-(whole apoB) antibody, and
   - detection of the presence of the particles by a suitable technique, in particular an enzyme immunoassay (EIA), a radioimmunoassay (RIA), an agglutination reaction or a fluorometric measurement.

2. Method according to Claim 1, characterized in that the said first antibody is immobilized on a suitable solid support, in particular beads or microtitration plates.

3. Method according to Claim 1 or Claim 2, characterized in that the second antibody is a polyclonal anti-apoB antibody.

4. Method according to Claim 1 or Claim 2, characterized in that the second antibody is a monoclonal anti-apoB antibody.

5. Method according to Claim 1 or Claim 2, characterized in that the said second antibody is an oligoclonal anti-apoB antibody.

6. Method according to any one of Claims 1 to 5, characterized in that the said second antibody is labelled by any suitable technique known per se, in particular using a labelling product chosen from the group comprising enzymes, fluorescent substances, radioactive substances or suitable latex beads.

7. Anti-Lp(a) antibodies, characterized in that they consist of monoclonal antibodies specific for apoprotein(a) (apo(a)), possessing a low affinity for plasminogen, expressed by their dissociation constant $K_D$ of between $0.45 \times 10^{-7}$M and $0.47 \times 10^{-7}$M, and a high affinity for apoprotein(a), expressed by their dissociation constant $K_D$ of between $0.87 \times 10^{-11}$M and $0.32 \times 10^{-10}$M.

8. Antibodies according to Claim 7, characterized in that they are obtained by cloning hybridomas resulting from the fusion of suitable myeloma cells and murine spleen cells immunized with an antigen consisting of an Lp(a) of human origin, employing a suitable cloning technique, and by selecting the hybrids that produce monoclonal antibodies specific for apoprotein(a) (apo(a)), possessing a low affinity for plasminogen, expressed by their dissociation constant $K_D$ of between $0.45 \times 10^{-7}$M and $0.47 \times 10^{-7}$M, and a high affinity for apoprotein(a), expressed by their dissociation constant $K_D$ of between $0.87 \times 10^{-11}$M and $0.32 \times 10^{-10}$M, and in that the said monoclonal antibodies are immunoglobulins of the $IgG_1$ type.

9. Application of the assay method according to any one of Claims 1 to 6 to the detection of atherosclerosis.

10. Ready-to-use kit or outfit for carrying out the method of assay of lipoprotein particles according to any one of Claims 1 to 6 and the application of the said method to the detection of atherosclerosis according to Claim 9, characterized in that it comprises:
- suitable doses of a first antibody chosen from the group consisting of monoclonal and oligoclonal antibodies directed against apolipoprotein(a);
- suitable doses of a second, suitably labelled anti-(whole apoB) antibody;
- a standard serum;
- appropriate amounts of suitable buffers for carrying out the said detection; and
- where appropriate, means of visualization of the presence of lipoprotein particles.

11. Kit according to Claim 10, characterized in that the first antibody is immobilized on a suitable solid support, in particular beads or microtitration plates .

12. Kit according to Claim 10, characterized in that the said first, monoclonal antibody is an antibody according to Claim 7 or Claim 8.

13. Kit according to any one of Claims 10 to 12, characterized in that the second antibody is a polyclonal anti-(whole apoB) antibody.

14. Kit according to any one of Claims 10 to 12, characterized in that the second antibody is a monoclonal anti-(whole apoB) antibody.

15. Kit according to any one of Claims 10 to 12, characterized in that the second antibody is an oligoclonal anti-(whole apoB) antibody.

16. Kit according to any one of Claims 10 to 15, characterized in that the second antibody is coupled to a suitable enzyme system.

17. Kit according to Claim 16, characterized in that, when the second antibody is coupled to an enzyme system, the said kit comprises a suitable amount of appropriate substrate as a means of visualization of the presence of lipoprotein particles.

18. Kit according to any one of Claims 10 to 15, characterized in that the second antibody is coupled to a suitable fluorescent substance.

19. Kit according to any one of Claims 10 to 15, characterized in that the second antibody is coupled to latex beads.

**Claims for the following Contracting States : ES, GR**

1. Method of immunometric assay of lipoprotein particles containing both apolipoprotein (a) (apo(a)) and apolipoprotein B (apoB), in a biological fluid, characterized in that it comprises:
- preparation of a sample of biological fluid at a dilution $\geqq$ 1/40;
- bringing the said diluted sample into contact with two different antibodies, namely a first antibody chosen from the group consisting of monoclonal or oligoclonal anti-apo(a) antibodies possessing a low affinity for plasminogen, expressed by their dissociation constant $K_D$ of between $0.45 \times 10^{-7}$M and $0.47 \times 10^{-7}$M, and a high affinity for apoprotein(a), expressed by their dissociation constant $K_D$ of between $0.87 \times 10^{-11}$M and $0.32 \times 10^{-10}$M, which antibodies capture the lipoprotein particle to be assayed, and a second, suitably labelled anti-(whole apoB) antibody, and
- detection of the presence of the particles by a suitable technique, in particular an enzyme immunoassay (EIA), a radioimmunoassay (RIA), an agglutination reaction or a fluorometric measurement.

2. Method according to Claim 1, characterized in that the said first antibody is immobilized on a suitable solid support, in particular beads or microtitration plates.

3. Method according to Claim 1 or Claim 2, characterized in that the second antibody is a polyclonal anti-apoB antibody.

4. Method according to Claim 1 or Claim 2, characterized in that the second antibody is a monoclonal anti-apoB antibody.

5. Method according to Claim 1 or Claim 2, characterized in that the said second antibody is an oligoclonal anti-apoB antibody.

6. Method according to any one of Claims 1 to 5, characterized in that the said second antibody is labelled by any suitable technique known per se, in particular using a labelling product chosen from the group comprising enzymes, fluorescent substances, radioactive substances or suitable latex beads.

7. Method for preparing anti-Lp(a) antibodies, characterized in that they are obtained by suitable immunization with an antigen consisting of an Lp(a) of human origin, which antibodies consist of monoclonal antibodies specific for apoprotein(a) (apo(a)), possessing a low affinity for plasminogen, expressed by their dissociation constant $K_D$ of between $0.45 \times 10^{-7}$M and $0.47 \times 10^{-7}$M, and a high affinity for apoprotein(a), expressed by their dissociation constant $K_D$ of between $0.87 \times 10^{-11}$M and $0.32 \times 10^{-10}$M.

8. Method for preparing the antibodies according to Claim 7, characterized in that they are obtained by cloning hybridomas resulting from the fusion of suitable myeloma cells and murine spleen cells immunized with an antigen consisting of an Lp(a) of human origin, employing a suitable cloning technique, and by selecting the hybrids that produce monoclonal antibodies specific for apoprotein(a) (apo(a)), possessing a low affinity for plasminogen, expressed by their dissociation constant $K_D$ of between $0.45 \times 10^{-7}$M and $0.47 \times 10^{-7}$M, and a high affinity for apoprotein(a), expressed by their dissociation constant $K_D$ of between $0.87 \times 10^{-11}$M and $0.32 \times 10^{-10}$M, and in that the said monoclonal antibodies are immunoglobulins of the $IgG_1$ type.

9. Application of the assay method according to any one of Claims 1 to 6 to the detection of atherosclerosis.

10. Ready-to-use kit or outfit for carrying out the method of assay of lipoprotein particles according to any one of Claims 1 to 6 and the application of the said method to the detection of atherosclerosis according to Claim 9, characterized in that it comprises:
    - suitable doses of a first antibody chosen from the group consisting of monoclonal and oligoclonal antibodies directed against apolipoprotein(a);
    - suitable doses of a second, suitably labelled anti-(whole apoB) antibody;
    - a standard serum;
    - appropriate amounts of suitable buffers for carrying out the said detection; and
    - where appropriate, means of visualization of the presence of lipoprotein particles.

11. Kit according to Claim 10, characterized in that the first antibody is immobilized on a suitable solid support, in particular beads or microtitration plates .

12. Kit according to Claim 10, characterized in that the said first, monoclonal antibody is an antibody according to Claim 7 or Claim 8.

13. Kit according to any one of Claims 10 to 12, characterized in that the second antibody is a polyclonal anti-(whole apoB) antibody.

14. Kit according to any one of Claims 10 to 12, characterized in that the second antibody is a monoclonal anti-(whole apoB) antibody.

15. Kit according to any one of Claims 10 to 12, characterized in that the second antibody is an oligoclonal anti-(whole apoB) antibody.

16. Kit according to any one of Claims 10 to 15, characterized in that the second antibody is coupled to a suitable enzyme system.

17. Kit according to Claim 16, characterized in that, when the second antibody is coupled to an enzyme system, the said kit comprises a suitable amount of appropriate substrate as a means of visualization of the presence of lipoprotein particles.

18. Kit according to any one of Claims 10 to 15, characterized in that the second antibody is coupled to a suitable fluorescent substance.

19. Kit according to any one of Claims 10 to 15, characterized in that the second antibody is coupled to latex beads.

FIG.1

FIG.2

FIG.3

FIG.4

FIG.5